(19) Europäisches Patentamt
European Patent Office
Office européen des brevets

(11) EP 1 880 730 A1

(12) EUROPEAN PATENT APPLICATION
published in accordance with Art. 153(4) EPC

(43) Date of publication:
**23.01.2008 Bulletin 2008/04**

(21) Application number: **06722399.0**

(22) Date of filing: **24.04.2006**

(51) Int Cl.:
***A61K 38/17*** *(2006.01)* ***A61P 1/14*** *(2006.01)*
***A61P 3/04*** *(2006.01)* ***A61P 25/04*** *(2006.01)*

(86) International application number:
**PCT/CN2006/000772**

(87) International publication number:
**WO 2006/111103 (26.10.2006 Gazette 2006/43)**

(84) Designated Contracting States:
**CH DE ES FR GB IT LI**

(30) Priority: **22.04.2005 CN 200510025323**

(71) Applicants:
- **Shanghai Institutes for Biological sciences, Chinese Academy of Sciences Shanghai 200031 (CN)**
- **F. Hoffmann-La Roche AG 4070 Basel (CH)**

(72) Inventors:
- **JIN, Meilei 200233 Shanghai (CN)**
- **PENG, Yun 200233 Shanghai (CN)**
- **ZOU, Hong 200233 Shanghai (CN)**
- **ZHAO, Guoping 200233 Shanghai (CN)**
- **ZHOU, Xuedong 200233 Shanghai (CN)**
- **CHUA, Anne, O. Wayne, NJ 07470 (US)**
- **GOODNOW, Robert, A. Gillette, NJ 07933 (US)**
- **GUBLER, Ulrich, A. Glen Ridge, NJ 07028 (US)**
- **HILTON, Holly Kearny, NJ 07032 (US)**
- **MARK, David, Fu-Chi West Windsor, NJ 08550 (US)**
- **MARTIN, Mitchell, Lee Verona, NJ 07044 (US)**
- **ROSINSKI, James, Andrew Nutley, NJ 07110 (US)**

(74) Representative: **Susanetto, Carlo et al CANTALUPPI & PARTNERS S.r.l. Via Matteotti, 26 35137 Padova (IT)**

# (54) FUNCTIONS AND USES OF GPR39 GENE IN MAMMALIAN CENTRAL NERVOUS SYSTEM

(57) The present invention provides mammalian GPR39 gene, its coded products, and the uses in regulating appetite and pain sensitivity. A pharmaceutical composition and a health product comprising GPR39 protein are also provided. The health product and the pharmaceutical composition for suppressing appetite or decreasing pain sensitivity comprise a safe and efficient amount of antagonists of mammalian GPR39 protein (for example, 0.01-99%) and a bromatologically or pharmaceutically acceptable carrier in a suitable amount (for example 1-99.99wt%).



Printed by Jouve, 75001 PARIS (FR)

## Description

### Technical Field

The present invention relates to biological field, especially to the uses of GPR39 gene in mammals and the coding products thereof in adjusting and controlling their appetite and pain feeling.

### Background Art

Appetite is the appetence of animal for taking food and water. Appetite is closely relevant to obesity. Many patients suffered from adiposis possess strong appetite. In addition, somebody, such as patients suffered from chronic disease, have low appetite. It tends to result in malnutrition. Therefore, people have kept on studying the method for adjusting and controlling appetite for many years. Furthermore, the cognition on pain feeling is limited, too.

GPR39 gene is a G-protein coupling receptor. According to presumption based on biological information, GPR39 protein, being consisted of 453 amino acids, comprises 7 trans-membrane regions having growth hormone secretagogue (GHSR). The homology between GPR39 gene and the other genes is low. GPR39 gene only has certain homology with growth hormone secretagogue (GHSR) and neurotensin receptor I (NTR1) (27% and 32% respectively). The fluorescence hybridization in situ shows that this gene is positioned at chromosome 2q21-q22[McKee KK, Tan CP, Palyha OC, Liu J, Feighner SD, Hreniuk DL, Smith RG, Howard AD, Van der Ploeg LH. Cloning and characterization of two human G protein-coupled receptor genes (GPR38 and GPR39) related to the growth hormone secretagogue and neurotensin receptors Genomics. 1997 Dec 15;46(3):426-34].

Cell experiments confirm that GPR39 activates downstream molecules in low level by means of inositol phosphate and CRE path, but activates downstream signals in continuous way and high level by means of SRE. [Birgitte Holstl, Nicholas D.Holliday2, Anders Bach1, Christian E.Elling3, Helen M.Cox2& Thue W.Schwartz1,3 Common structural basis for constitutive activity of the ghrelin receptor family J Biol Chem. 2004 Sep 21]. However, the function of this gene, especially the function thereof on integral animals has been unclear.

Up to the present, little is learnt about various of proteins involving or affecting appetite and pain feeling. Therefore, it is desirable to find novel proteins associated with appetite and pain feeling so as to develop medicaments for the control of appetite and pain feeling.

### Contents of the Invention

Therefore, one object of the invention is to provide an appetite-relevant protein named GPR39 protein and its use in regulation of appetite.

Another object of the invention is to provide a pharmaceutical composition or a health product containing a GPR39 protein or an agonist or antagonist thereof.

In the first aspect, the invention provides a use of a mammalian GPR39 protein or an agonist or antagonist thereof in manufacture of a health product or a pharmaceutical composition for regulating appetite or pain sensitivity in a mammal.

In one embodiment of the invention, the said pharmaceutical composition comprises a safe and effective amount of the mammalian GPR39 protein and a pharmaceutically acceptable carrier. In an alternative embodiment, the said pharmaceutical composition comprises a safe and effective amount of an antagonist of the GPR39 protein and a pharmaceutically acceptable carrier.

In another embodiment, the said GPR39 protein is of an animal origin selected from the group consisting of human being, rat and mouse. Preferably, the said GPR39 protein has an amino acid sequence as set forth in SEQ ID NO: 2, 4 or 6.

In another embodiment, the said pharmaceutical composition comprises, based on the total weight of the composition, 0.01-90wt% of the GPR39 protein.

In another embodiment, the said health product or the said pharmaceutical composition is in a form selected from the group consisting of tablet, capsule, granules or solution.

In the second aspect, the invention provides a health product or a pharmaceutical composition for suppressing appetite or decreasing pain sensitivity, comprising a safe and effective amount (e.g., 0.01-99%) of an antagonist of a mammalian GRP39 protein and an bromatologically or pharmaceutically acceptable carrier in a suitable amount such as 1-99.99wt%.

Preferably, the said antagonist is an antibody to a GRP39 protein, an antisense nucleotide or an iRNA of a GRP39 gene.

In the third aspect, the present invention provides a method of screening for a candidate agent for suppressing appetite or decreasing pain sensitivity, which comprises the steps of:

a) producing a GPR39 protein-expressing cell line by inserting a cDNA of a GPR39 gene into an expression vector and transfecting a mammalian cell line with the obtained expression vector;
b) adding a test compound into a culture of the GPR39 protein-expressing cell line obtained in step a), and detecting changes in the expression of GPR39 protein,

wherein a compound that inhibits increase of the expression of GPR39 protein is identified as a candidate agent for suppressing appetite or decreasing pain sensitivity.

In a preferred embodiment, the GPR39 protein has an amino acid sequence as set forth in SEQ ID NO: 2, 4 or 6.

In the forth aspect, the invention provides a method for screening for a candidate agent for enhancing appetite or pain sensitivity, comprising the steps of:

a) producing a GPR39 protein-expressing cell line by inserting a cDNA of a GPR39 gene into an expression vector and transfecting a mammalian cell line with the obtained expression vector;
b) adding a test compound into a culture of the GPR39 protein-expressing cell line obtained in step a), and detecting changes in the expression of GPR39 protein,

wherein a compound that enhances increase of the expression GPR protein expression is identified as a candidate agent for enhancing appetite or pain sensitivity.

In a preferred embodiment, the GPR39 protein has an amino acid sequence as set forth in SEQ ID NO: 2, 4 or 6.

## Brief Description of the Figures

Figure 1 shows the comparison on food intake between the test group of BALB/C mice and the control groups. The x-axis represents the days, and the y-axis represents the mean of daily food intake in each group consisting of 10 mice. The injection to the three groups of animals started at day 3. As shown, the antisense nucleotide group ("antisense group") showed a significant decrease in food intake at day 6 in comparison to the saline group. As shown by the t-test, the difference was significant ($P<0.05$, antisense group vs. saline group).

Figure 2 shows the comparison on food intake between the test group of ICR mice and the control groups. The x-axis represents the days, and the y-axis represents the mean of daily food intake in each group consisting of 10 mice. The injection to the animals started at Day 3 for all three groups. As shown, the antisense group showed a significant decrease in food intake at day 6 in comparison to the control group. As shown by the t-test, the differences were significant ($P<0.05$, antisense group vs. saline group, $p<0.01$, antisense group vs. missense group).

Figure 3 shows the means of apparent digestibility in the three groups of BALB/c mice. T test did not show significant differences among the groups. The apparent digestibility was calculated by the formula: apparent digestibility = (N content in protein of daily intake - N content in feces of daily excretion)/ N content in protein of daily intake $\times$ 100%.

Figure 4 shows the comparison on rearing behavior between the test group of ICR mice and the control group. The y-axis represents the mean of rearing numbers in each groups. The time of the Open Field Behavior test was set to be 1 hour. The data of the second half hour was statically analyzed. As shown by the t-test, the difference between the antisense group and either of the control groups was significant ($P<0.05$, antisense vs. saline; $P<0.05$, antisense vs. missense).

Figure 5 shows the results in the Tail Flick tests with the ICR mice. The x-axis represents the three different groups, and the y-axis represents the mean tail flicking latency in each group. As shown by the t-test, the difference between the antisense group and either of the control groups was significant ($P<0.05$, antisense vs. saline; $P<0.05$, antisense vs. missense; n=10).

## Detailed Description of the Invention

Extensive and intensive studies on the functions of the GPR 39 gene in various animal behavior models have been done by the present inventors. Surprisingly, it is found that GPR 39 is involved in the regulation of food intake and pain sensitivity. The present invention is completed based on the new finding.

Specifically, antisense and missense nucleic acid of a GPR39 gene were designed and synthesized using the bio-information analysis. The nucleotides were injected into the lateral ventricles of mouse brain at predetermined doses using a Hamilton micro-syringe. Each experiment included three groups, i.e., the antisense group, the missense control group and the saline control group. The differences between the test group and the control groups in the animal behavior studies were monitored. The said behavior tests include the Open Field Behavior test, the Hole-Board test, the Tail Flick test and the Step-down test.

The results show that the food intake in the test group was significantly less than the control; in the Open Field Behavior test, the rearing number in the test group was significantly higher than the control groups; and in the Tail Flick test, the tail flick latency time in the test group is significantly longer than the control. All these suggest that inhibition of GPR39 gene results in a suppression of appetite, an increasing in rearing, and insensitivity to pain stimuli in mouse. In other words, when normally expressed, this gene contributes to the regulation on food intake, mobility and pain sensitivity.

As used in the present invention, the terms "GPR39 protein" and "GPR39 polypeptide" are interchangeable, both referring to a polypeptide that is expressed in various systems including the nerve system and has an amino acid sequence at least 80%, preferably 85%, more preferably 90% identical or homologous to the GPR39 sequence in human, rat or mouse. Also, active fragments, conservative polypeptides and functional derivatives of GPR39 are also useful in the present invention.

The DNA sequence and the amino acid sequence of the mouse GPR39 protein were shown in SEQ ID NOs: 1 and 2. (GenBank accession number: BC 085285)

The DNA sequence and the amino acid sequence of the human GPR39 protein were shown in SEQ ID NOs: 3 and 4. (GenBank accession number: NM 001508)

The DNA sequence and the amino acid sequence of the rat GPR39 protein were shown in SEQ ID NOs:5 and 6. (GenBank accession number: XM 222578)

As shown in the homology comparison, the human GPR 39 protein is highly homologous to the mouse counterpart with an identity of at least 82%. The structure and the distribution of the mouse GPR 39 protein suggest that the GPR39 protein regulates the appetite, mobility and pain sensitivity. Accordingly, the human GPR39 protein is also expected to contribute to the appetite-regulation and pain sensitivity-regulation.

The full-length GPR39 nucleotide sequence or its fragment according to the invention can be prepared by PCR amplification, recombinant method and synthetic method. For PCR amplification, primers can be designed according to the known sequences, especially the Open Reading Frame (ORF) of the human GPR39 and the mouse GPR39, and the templates may be cDNA library commercially available or prepared by conventional method in the art. Then, the desired sequence is obtained by amplification. If the sequence is long, two or more rounds of PCR amplifications may be needed, and then the products from each rounds are appropriately linked into the correct sequence.

Once the sequence is obtained, a great amount of the sequences can be produced by recombinant methods. Usually, said sequence is cloned into a vector which is then transformed into a host cell. Then the sequence is recovered from the proliferated host cells using conventional techniques.

Further, the sequences, especially the short ones, can be produced by synthesis. Typically, several small fragments are synthesized, and then linked together into a long sequence.

Currently, the DNA sequences encoding the proteins and their fragments and derivatives of the present invention can be fully synthesized. Then, the synthesized DNA sequence may be introduced into various DNA molecules (e.g., vectors) and cells known in the art. Additionally, mutation may be introduced into the protein sequence by chemical synthesis.

The GPR39 protein of the invention can be produced by introducing an encoding sequence of the said protein into a suitable host cell, incubating the cells under suitable condition for expression of the GRP39 protein by the cells and then isolating and purifying the protein from the culture. The said encoding sequence of GPR39 can be directly introduced into the said host cells or indirectly introduced in form of a vector containing the said encoding sequence.

The GPR39 protein or polypeptide of the invention are useful in various applications including but not limited to: curing disorders such as appetite loss due to impaired or lack of GPR39 functions (wherein, the said protein or polypeptide may directly be used as a therapeutic agent), and screening for antibodies, polypeptides or other ligands that promote the functions of GPR39. The expressed recombinant GPR39 protein can be used to screen polypeptide libraries for a therapeutically valuable polypeptide molecule that activates the functions of GPR39 protein.

In another aspect, the invention also includes polyclonal and monoclonal antibodies, preferably monoclonal antibodies, which are specific for a polypeptides encoded by a GPR39 DNA or fragments thereof. By "specific", it means that the antibody binds to a GPR39 gene product or a fragment thereof. Preferably, the antibody binds to a GPR39 gene product or a fragment thereof, while substantially not recognizing or binding to irrelevant antigenic molecules. As in the present invention, the antibodies include the molecules that bind to and block the GPR39 proteins and also the antibodies that do not interfere the functions of GPR39 proteins.

The present invention includes not only the intact monoclonal or polyclonal antibodies but also the immunologically-active antibody fragments such as the Fab' or the $(Fab)_2$ fragments, the heavy chains, the light chains and the chimeric antibodies such as a chimeric antibody comprising the binding specificity of a murine origin in a frame of a human origin.

The antibodies in the present invention can be prepared by various techniques known in the art. For example, a purified GPR39 gene product or its antigenic fragments can be administrated to an animal to induce the production of polyclonal antibodies. Similarly, cells expressing GPR39 proteins or their antigenic fragments can be used to immunize animals to produce antibodies. The monoclonal antibodies of the invention can be prepared using the hybridoma technique (Kohler et al., Eur. J. Immunol., 6:292, 1976; Hammerling et al., In Monoclonal Antibodies and T Cell Hybridomas, Elsevier, N.Y., 1981). The antibodies of the invention can be obtained using fragments or functional domains of the GPR39 gene product of a human, rat or mouse origin and conventional immunological techniques. The said fragments or domains can be recombinantly produced or prepared on a polypeptide synthesizer. The antibodies binding to an unmodified form of a GPR39 gene product can be produced by immunizing animals with the GPR39 gene product from prokaryotic cells (e.g., E. coli), while the antibodies binding to a post-translationally modified form (e.g., glycosylated or phophorylated form) can be acquired by immunizing the animals with a gene product from eukaryotic cells (e.g., yeast or insect cells).

The antibody against the GPR39 protein can be used in immunohistochemistry to detect the GPR39 protein in a biopsy specimen.

The polyclonal antibodies may be produced by immunizing the animals such as rabbit and goat with a GPR39 protein or polypeptide. An adjuvant, including but not limited to the Freund's adjuvant, can be used to enhance the immune response.

The substances that interact with the GPR39 protein, including inhibitors, agonists and antagonists, can be obtained in screening processes using the protein of the invention. In a screening process, for example, a GPR39 protein may be added into a biology assay, and the change in the interaction between the protein and its receptor in the presence of a test compound was detected to determine whether the compound is a antagonist. Additionally or alternatively, a test compound may be administered to an animal in combination with the GPR39 protein, and the changes in appetite and pain sensitivity were detected to determine whether the compound is a GPR39 agonist or antagonist.

Further, the cDNA of the GPR39 gene may be inserted into a suitable vector to transfect a mammalian animal cell line to produce a cell line characterized in high expression of GPR39 protein. The expressed GPR39 protein in the obtained cell line can then be utilized as a target in search for an agent activating or inhibiting the GPR39 protein. A test compound can be added into a culture of the said line to see if it changes the GPR39 expression. A compound promoting the GPR39 expression may then be identified as a candidate agent for treating loss of appetite or insensitivity to pains, while one inhibiting the expression may then be identified as a candidate agent for suppressing appetite or algesia.

The GPR39 protein, antibody, inhibitor, agonist or antagonist according to the invention, when administrated in therapy, will provide different effects. Usually, these substances are formulated with a non-toxic, inert and pharmaceutically acceptable aqueous carrier at a suitable pH depending on the nature of the components in the formula and the nature of the diseases to be treated, typically about pH 5 to 8, preferably about pH 6 to 8. The formulated pharmaceutical composition then can be administrated via intramuscular, intravenous, subcutaneous, oral or topical routs or any other suitable routs that can be readily determined by a physician.

The normal GPR39 polypeptide can be directly used for curing disorders such as loss of appetite or insensitivity to pains. Also, additional agents for treating loss of appetite can be used in combination with the GPR39 protein of the invention.

The invention also provides a pharmaceutical composition comprising a safe and effective amount of GPR39 protein in combination with a pharmaceutically acceptable carrier or excipient. The suitable carriers include but are not limited to saline, buffer solution, glucose, water, glycerin, ethanol or the combination thereof. The pharmaceutical formulation should be adapted to administration routes. The pharmaceutical composition of the invention may be formulated into an injectable form with a suitable aqueous solution such as a physiological saline or a solution containing glucose and other excipient(s) by the standard techniques. The pharmaceutical compositions such as the injectable solution, solution, tablets or capsules are preferably prepared under sterile condition. The active ingredient is administrated in a therapeutically effective amount, e.g., from about 0.1ug to 5mg per kg body weight per day. Moreover, the polypeptide of the invention can be used in combination with other therapeutic agents.

By using the pharmaceutical composition, a safe and effective amount of the GPR39 protein or its antagonist or agonist is administrated to a mammal. Typically, the safe and effective amount is at least about 0.1 ug/kg body weight while not more than about 10 mg/kg body weight in most cases. Preferably, the amount may be about 0.1-100ug/kg body weight. Of course, a precise amount depends upon various factors including the routes of administration and the health status of the recipient, etc., and can be readily determined by a clinician.

Polynucleotides of GPR39 may also be used in therapies. For example, gene therapy can be used to treat abnormal cell proliferation, development or metabolism due to impaired expression of GPR39 or expression of abnormal or inactive GPR39 proteins. The construction of recombinant viral vectors harboring GPR39 gene have been reported (Sambrook, et al.). A recombinant human GPR39 gene can be packaged into a liposome, which is then transferred into a suitable cell.

The methods for introducing a polynucleotide into tissues or cells include direct injection of the polynucleotide into a tissue in body and introduction of the polynucleotide into cells with a vector (e.g., a virus, a phage, or a plasmid ) *in vitro* followed by transplantation the treated cells into body.

The invention further provides diagnostic assays for quantitative and *in situ* measurement of GPR39 protein level. Such assays are well known in the art, which include, for example, FISH assay and radioimmunoassay. The GPR39 protein level determined in the said assays may explain the roles the GPR39 protein plays in a given disease or give a diagnosis of a GPR39 protein-associated disease.

A method of detecting the presence of GPR39 protein in a sample utilizes an antibody specific to a GPR39 protein, which comprises the steps of: contacting the sample with the antibody specific to the GPR39 protein, detecting the formation of an antibody complex which indicates the presence of the GPR39 protein in the sample.

The polynucleotides encoding GPR39 protein can be used in the diagnosis and treatment of GPR39 protein-associated diseases. For diagnosis, the polynucleotide encoding GPR39 can be used to determine whether GPR39 is expressed, or, in the case of a disease condition, whether the expression of GPR39 is abnormal. For example, a GPR39 DNA sequence can be used in the hybridization with a biopsy sample to detect an abnormal expression of GPR39. The hybridization may be, for example, a Southern blotting, Northern blotting and *in situ* blotting, all being widely known and sophisticated techniques. The corresponding kits have been commercially available. A polynucleotide of the invention or a part of it may be used as a probe to be fixed on a microarray or DNA chip for analysis of differential expression of genes in tissues and genetic diagnosis. GPR39 specific primers can be used in RNA-polymerase chain reaction and *in vitro* amplification to detect the transcripts of GPR39.

Detection of mutations in the GPR39 gene may also be used for diagnosis of a GPR39-associated disease. The mutation may be a site-specific mutation, shift, deletion, rearrangement or other abnormalities. The mutation can be detected by, for example, Southern blotting, DNA sequencing, PCR and *in situ* hybridization and other techniques known in the art. Since mutation in gene may change the expression of the encoded protein, Northern blotting and Western blotting may also be useful in detecting mutation in gene.

The GPR39 protein of the invention is useful not only in treating loss of appetite but also in improving appetite in a healthy subject. The GPR39 protein antagonist can be used to depress the appetite in a healthy subject. Accordingly, the present invention also provides an appetite-enhancing health care product, which comprises a mammalian GPR39 protein or its functional fragment, derivative or agonist. Also, the invention provides an appetite-reducing health care product, which comprises an antagonist to mammalian GPR39 protein. The health care products according to the invention can be prepared by following the conventional practices in the art. For example, they can be prepared by mixing the mammalian GPR39 protein or its functional fragment (or its agonist or antagonist) with a suitable diluent, food stuff, etc. Preferably, the said product is in form of a tablet, granules or any other suitable oral formulation.

Additionally, the invention provides a method for treating loss of appetite, wherein the said method comprises the step of administrating to a patient in need of the treatment a safe and effective amount of normal GPR39 protein or its functional fragment or agonist.

Additionally, the invention provides a method for suppressing appetite in a subject, wherein the said method comprises the step of administrating to a patient in need of the treatment a safe and effective amount of an antagonist to GPR39 protein.

Additionally, the invention provides a method for regulating appetite and pain sensitivity in a mammal, wherein the said method comprises the step of administrating to a patient in need of the treatment a safe and effective amount of GPR39 protein, its agonist or its antagonist.

More features and advantages can be seen from the examples detailed as follows. It should be understood that the examples are only provided for illustration without limiting the invention in any sense. All the experiments were carried out under standard conditions such as those taught in the Molecular Cloning: A Laboratory Manual (Sambrook et al., New York: Cold Spring Harbor Laboratory Press, 1989) or under conditions as suggested by the manufacturer.

**1.Materials and Equipments**:

1. 1. Agents

1.1.1. Antisense nucleic acids: The antisense nucleic acid was designed by the Institute of Biochemistry and Cell Biology, Shanghai Institute for Biological Sciences, Chinese Academy of Sciences, and was synthesized by Shanghai Genebase Gene-Tech Co., Ltd.

The sequence of the antisense nucleic acid is 5'-TCG GAT CTG ATT GGG CAT -3'(SEQ ID NO:7), and

The sequence of the missense nucleic acid is 5'-TTG GGT CTG ATC GGA GAT-3'(SEQ ID NO:8).

1.1.2. Pentobarbital Sodium, purchased from Guangzhou South Huabo Company.

1.2. Instruments:

1.2.1. Stainless steel metabolic chamber, homemade
1.2.3. Hamilton micro-syringe

1.2.3. Open Field Behavior test system (Flax Field System ), a product of the SD company, USA
1.2.4. Infrared Tail Flick Ltency Meter (TFL meter), a product of the Stoelting Company, USA
1.2.5. Hole-board instrument, a product of the Stoelting Company, USA
1.2.6. Step-down monitor, homemade

1.3. Experiment Animals:

1.3.1. Inbred BALB/c mouse, male, 20-22g weight, SPF grade, purchased from Shanghai Laboratory Animal Center, Chinese Academy of Sciences.
1.3.2. Cross-bred population ICR mouse, male, 20-22g weight, SPF grade, purchased from Shanghai Laboratory Animal Center, Chinese Academy of Sciences.

**Example 1**

**Investigation on the functions of GPR39**

In this example, the functions of GPR39 were studied using the antisense technique. The antisense technique is based on the complementary matching between the base pairs. The transcription and translation of a target gene may be blocked by using an antisense molecule specifically binding to the target gene or its mRNA transcript, and thereby decreasing or preventing the expression of the target protein. In this example, the antisense technique was used to temporarily decrease the GPR39 expression in mouse, which allowed the studies on the functions of the gene in metabolism and other behaviors.

**(a). Methods**:

**1. The design and synthesis of antisense, missense nucleic acids and the formulation of the solutions**

By using the bio-information techniques, the antisense and the missense nucleic acids were designed and synthesized for selected domains in the translation initiation region or other regions in the cDNA of the gene. The obtained antisense molecules and the missense molecules were respectively formulated into 0.5 μg/μl solutions in separate tubes, which were marked and stored in refrigerator at -20°C before use.

**2. Injection in Lateral Cerebral Ventricle**

The mouse were anaesthetized with 1 % Pentobarbital Sodium solution (120 μl per 20g body weight). The head of the animal was sterilized using 75% ethanol-dipped cotton balls. At the median line slightly rear to the cyc-balls, a section of 0.6-0.8mm was made to expose the skull. At 1 mm rear to the skull suture at one side to the median line, a hole was drilled by using needle, through which 4 μl of antisense solution (for the test group), saline (for the control) or missense solution (for the control) was slowly injected into the lateral cerebral ventricle using a Hamilton micro-syringe in about 2-3 minutes. Caution should be taken to avoid damage to the vasculars nearby. When the injection was finished, the needle was left for about 30 seconds, allowing the solution penetrating into the cerebral tissues, and then cautiously pulled out. Finally, the treated mice were put back into the metabolism chambers.

**3. Metabolism Study**

The experiments were carried out as previously reported ("Studies on four genes in mouse for their effects on food intake, water intake and protein metabolism", Ao, Hong ct al, Acta Laboratorium Animalis Scientia Sinica, 10(1): 10-15, 2002). Briefly, every experiment included 30 mice, which were divided into three groups being the test group, the antisense group and the missense group, each group consisting of 10 animals. Each stage in the experiment is detailed as follows.

**3.1. Adaptation:**

After being obtained from the Center, the mice were allowed to adapt to the breeding house for about two days. In the feeding chamber, the animals were let free access to food and water, and were kept under natural day light from 6:00 AM to 6:00 PM (12 hours of daylight).

### 3.2. Experiment:

In forenoon of Day 1, the mice after adaptation were randomly divided into three groups, 10 animals each. Every mouse was weighed and numbered and then put into the stainless steel metabolism chamber inside which a weighed feed box and a weighed water bottle were placed. The animals were let free to adapt to the chamber.

At the same time of D2, the mice were weighed. And, the feed box and the water bottle were weighed to calculate the food intake and the water intake. Then, the feed box and the water box were refilled and weighed. The feces were collected and weighed.

In forenoon of Day 3, the operations in Day 2 were repeated. The data were then input into a computer to examine the differences among the groups. When no significant difference was observed, the first intracerebral injection in lateral ventricle was done in the afternoon of the even day.

In the following four days, the operations as above were repeated everyday. That is, the animal, the feed box and the water bottle were weighted in the forenoon, and the intracerebral injection in lateral ventricle was done in the afternoon.

After the five continuous daily injections, the injection was stopped at Day 8 and Day 9. The animals, the feed box and the water bottle were weighed. The observation on the metabolism went on.

## 4. Behavioral Tests

### 4.1. Open Field Behavior:

The whole test system was divided into 8 wells. Every time the mouse cut the infrared in its movement, the meter would automatically record it, and then, have the data statistically summarized.

### 4.2. Tail Flick Latency Test:

In the test, the light intensity was set at 88. The mouse was wrapped with cotton cloth to expose the tail only, whereby the animal could be kept under capture without being tortured. The tail about 1.5cm in length from the tip was put on a hole above a heat radiation. Each test run three times, while the data from the latter two were used for statistic analysis.

### 4.3. Hole-Board test:

The animal was put in to the Hole-Board meter. Number of hole-seeking in 5 minutes were determined.

### 4.4. Step Down Test:

A Step Down training was run in the even day when the injections were finished. The mouse was put into the plastic Step-Down chamber and let adapt for about 1 minute. Then, the mouse was driven onto the step, and the board was electrified. When the mouse stepped down, it got an electric shock and retreated onto the step immediately. The animal who did not step down within 3 minutes after the shock was evaluated as trained competent and put back into the metabolism chamber. 24 hours later, the mouse was put back onto the step, and the board was not galvanized. The time between the animal being back onto the step and first stepping down was recorded. The test was repeated 48 hours later, and the time was recorded

## 5. Process of the Tests

On Day 1 and Day 2, the mice were allowed to adapt to the environment inside the metabolism chamber and the manners of feeding and watering. On Days 3, 4, 5, 6, 7, intracerebral injection in lateral ventricle was done on a daily basis. From Day 1 to Day 9, the metabolism of the animals were monitored. On Day 8, behavioral tests were run.

## 6. Statistic Analysis

Statistic analysis of the data was ran with the Prism software.

**(b) Results:**

**1. Changes In Food Intake In The Metabolism Experiment**

The comparison on food intake between the test group of BALB/C mice and the control groups was shown in figure 1. After receiving the intracerebral injection in lateral ventricle, mice in three groups all showed a decrease in food intake due to the anesthesia and the surgery. With the subsequent injections, the food intake went up in the control groups, while kept going down in the test group and reached the bottom on Day 5, which showed a significant difference from the control group receiving saline. After the fifth injection, the food intakes in three groups reverted to equivalent levels.

The comparison on food intake between the test group of ICR mice and the control groups was shown in figure 2. The injection started in all groups at Day 3. The antisense group showed a significant decrease in food intake at day 6 in comparison to the control groups. As shown by the t-test, the differences were significant ($P<0.05$, antisense group vs. saline group; $p<0.01$, antisense group vs. missense group).

**2. Apparent Digestibility**

The apparent digestibility was calculated by the formula:

$$\text{apparent digestibility} = (\text{N content in protein of daily intake} - \text{N content in feces of daily excretion}) / \text{N content in protein of daily intake} \times 100\%.$$

The results in the three groups of BALB/c mice were shown in figure 3. T test did not show significant differences among the groups.

**3. Behavioral Tests**

**3.1. Rearing Number**

The Open Field Behavior test was run after the 5 injections. The time of the test was set to be 1 hour, and the data of the second half hour was statically analyzed. The results obtained with the ICR mouse were shown in Figure 4. As shown by the t-test, the difference between the antisense group and either of the control groups was significant ($P<0.05$, antisense vs. saline; $P<0.05$, antisense vs. missense). The results shown that the rearing number in the test group is higher than in the control, indicating a higher mobility than the control.

**3.2.** Tail Flick test

The Tail Flick test was run immediately after the Open Field Behavior test. The results obtained with ICR mouse were shown in figure 5. The mice in the test group is less sensitive to pains, suggesting a decrease in pain sensitivity. As shown by the t-test, the difference between the antisense group and either of the control groups was significant ($P<0.05$, antisense vs. saline; $P<0.05$, antisense vs. missense).

**Discussion**

The results shown that, when the expression of GPR39 gene is down-regulated, the food intake by the mice in the test group significantly decreases, while the apparent digestibility does not significantly differs among the groups. This may suggest that GPR39 gene, when expressed at a normal level, enhance the appetite in mouse, while the over expression of which may lead to obesity.

Meanwhile, behavior tests showed that the GPR39 gene also contributes to the rearing behavior and pain sensitivity. Urine analysis was not done in the studies, which may slightly influence the statistic results of the metabolism tests.

In view of its relevancy to food-intake regulation as shown in the results, GPR39 shows promising value in development of weigh-controlling drugs and therapy of obesity. Medication to inhibit the expression of the gene or to antagonize its products will make it possible to reduce weight by appetite suppression. Up till now, the roles this gene plays in the pathway of regulation on food intake has not been fully understood, and its endogenous ligands has not been known. More work in cytology and molecular biology is yet needed.

**Example 2**

**Composition of GPR39 protein**

Recombinant GPR39 protein, lactose, Crospovidone (cross linking polyvinylpyrrolidone) (the International Special Product Compnay, "ISP"), aspartame (The NutraSweet Company) in the amounts as shown in the following table were mixed to homogeneity. The obtained mixture was sifted twice through a 40-mesh screen. 10 % starch slurry was added as appropriate to prepare a soft material. The soft material was extruded through a 20-mesh screen to give wet granules. The obtained wet granules were put into a dry enamel plate, and dried at 70°C for 2 hrs in a thermostatic blast oven. The dried granules were weighted. Magnesium stearate was added in an amount of 1wt% based on the weight of the dried granules. The obtained homogeneous mixture was pressed into 1000 tablets using the ZDY-1S mono-stroke tablet presser (Shanghai Far-East Pharmaceutical Equipment, Model ZDY-8) to provide oral disintegratable tablet of GPR39 protein.

| Ingredients | Amounts |
|---|---|
| GPR39 protein, gram | 0.5 |
| Lactose, gram | 150 |
| Crosspovidone, gram | 40 |
| Aspartame, gram | 5 |
| 10% Starch Slurry, gram | 10 |
| Magnesium stearate | 1wt% |

The obtained oral disintegratable tablets may be used as either a health product or a pharmaceutical agent to enhance food intake in patients suffering from appetite loss due to asthenia or in the population of anorexia. Two volunteers complaining appetite loss took the tablet on a basis of twice a day and one tablet once. A week later, the two volunteers reported enhancement in food intake.

All the references are incorporated by reference in their entirety. It should be understood that in view of the description in the above, the possible changes and modifications not going away from the spirits and concept of the invention will be obvious to one skilled in the art, and should all be contemplated as falling with the scope of the invention that is only defined by the claims as follows.

## Claims

1. Use of a mammalian GPR39 protein or an agonist or antagonist thereof in manufacture of a health product or a pharmaceutical composition for regulating appetite or pain sensitivity in a mammal.

2. The use according to claim 1, wherein the said pharmaceutical composition or health product comprises a safe and effective amount of the mammalian GPR39 protein and a pharmaceutically acceptable carrier; or
the said pharmaceutical composition or health product comprises a safe and effective amount of an antagonist of the mammalian GPR39 protein and a pharmaceutically acceptable carrier.

3. The use according to claim 1, wherein the GPR39 protein is of an animal origin selected from the group consisting of human being, rat and mice.

4. The use according to claim 1, wherein the pharmaceutical composition or health product comprises the GPR39 protein in an amount of 0.01-90 wt% based on the total weight of the composition.

5. The use according to claim 1, wherein the health product or pharmaceutical composition is in a form selected from the group consisting of tablet, capsule, granule or solution.

6. A health product or pharmaceutical composition for suppressing appetite or decreasing pain sensitivity, comprising a safe and effective amount of an antagonist of a mammalian GPR39 protein and a bromatologically or pharmaceutically acceptable carrier.

7. A method for screening for a candidate agent for suppressing appetite or decreasing pain sensitivity, comprising the steps of:

    a) producing a GPR39 protein-expressing cell line by inserting a cDNA of a GPR39 gene into an expression vector and transfecting a mammalian cell line with the obtained expression vector;
    b) adding a test compound into a culture of the GPR39 protein-expressing cell line obtained in step a), and detecting changes in the expression of GPR39 protein,

   wherein a compound that inhibits increase in the expression of GPR39 protein is identified as a candidate agent for suppressing appetite or decreasing pain sensitivity.

8. The use according to claim 7, wherein the GPR39 protein has an amino acid sequence as set forth in SEQ ID NO:

2, 4 or 6.

**9.** A method for screening for a candidate agent for enhancing appetite or pain sensitivity, comprising the steps of:

a) producing a GPR39 protein-expressing cell line by inserting a cDNA of a GPR39 gene into an expression vector and transfecting a mammalian cell line with the obtained expression vector;
b) adding a test compound into a culture of the GPR39 protein-expressing cell line obtained in step a), and detecting changes in the expression of GPR39 protein,

wherein a compound that enhances increase in the expression of GPR protein is identified as a candidate agent for enhancing appetite or pain sensitivity.

**10.** The use according to claim 9, wherein the GPR39 protein has an amino acid sequence as set forth in SEQ ID NO: 2, 4 or 6.

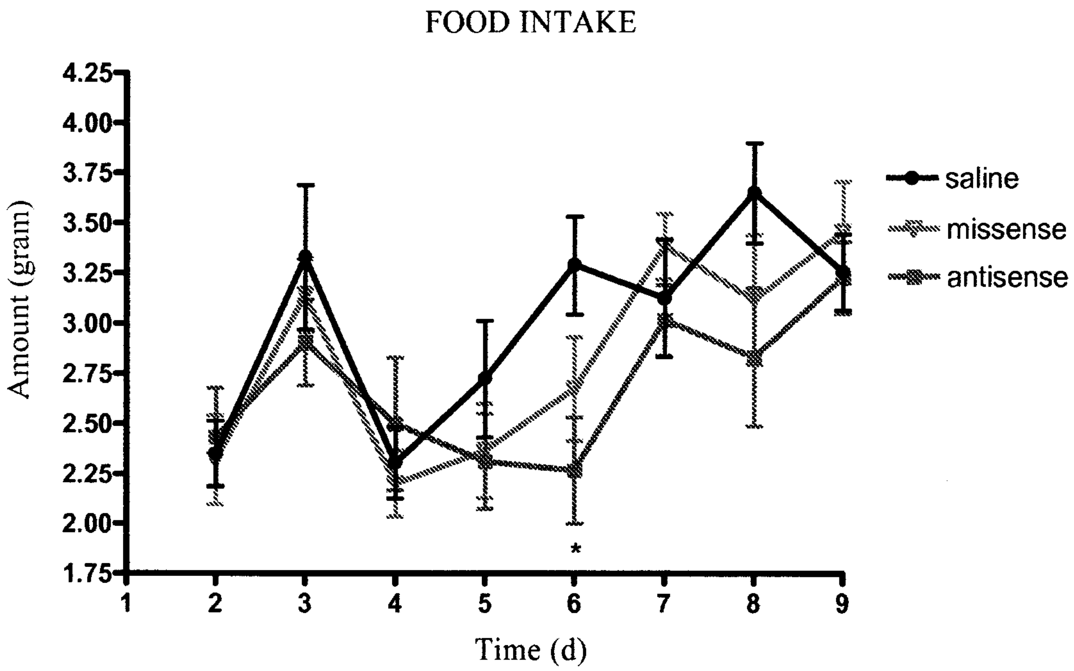
FOOD INTAKE
Amount (gram)
4.25
4.00
3.75
3.50
3.25
3.00
2.75
2.50
2.25
2.00
1.75
1 2 3 4 5 6 7 8 9
Time (d)
*
saline
missense
antisense

FIG. 1

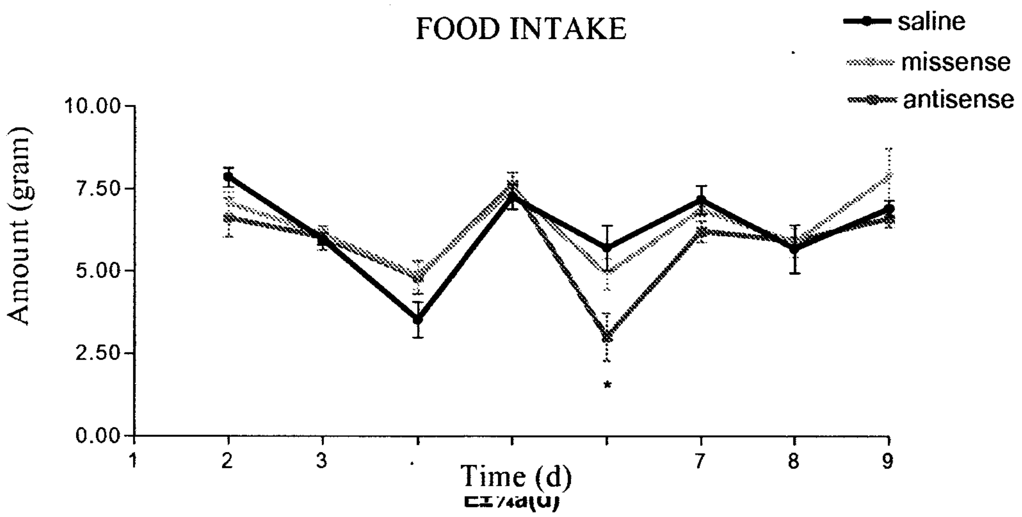
FOOD INTAKE
saline
missense
antisense
Amount (gram)
10.00
7.50
5.00
2.50
0.00
1 2 3 7 8 9
Time (d)
*

FIG. 2

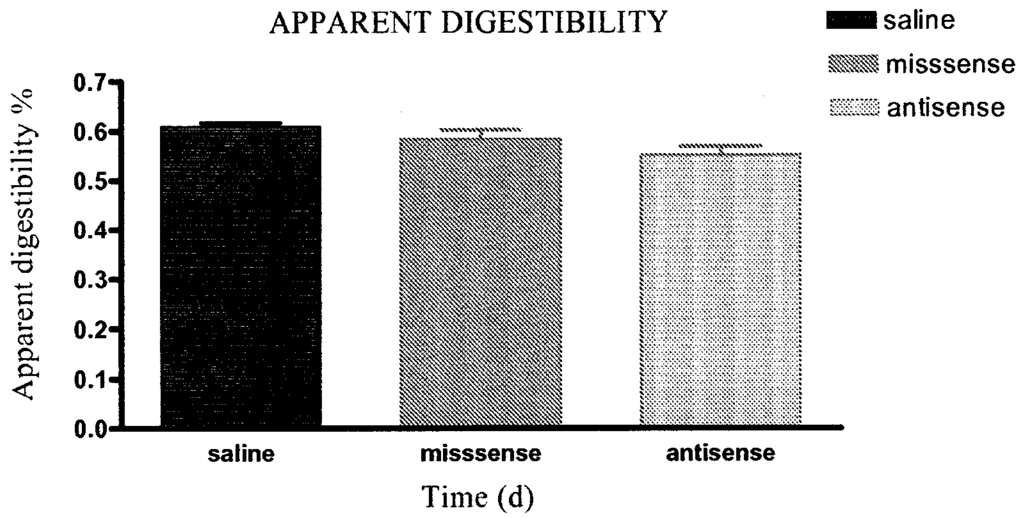
APPARENT DIGESTIBILITY
saline
misssense
antisense
Apparent digestibility %
0.7
0.6
0.5
0.4
0.3
0.2
0.1
0.0
saline
misssense
antisense
Time (d)

FIG. 3

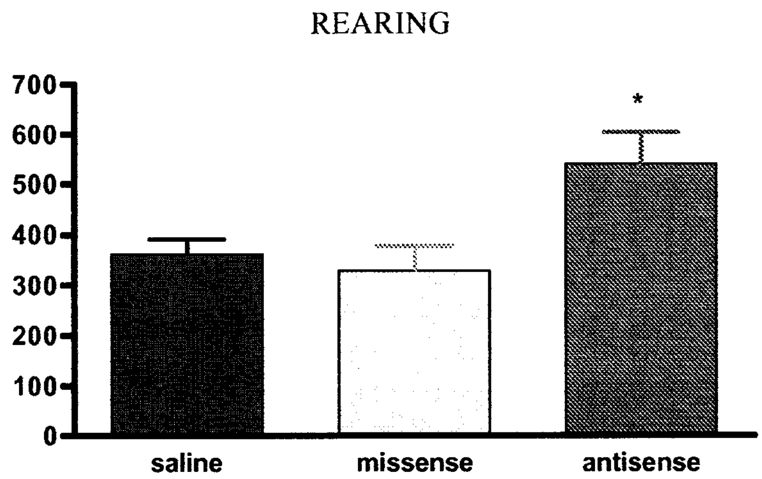
REARING
700
600
500
400
300
200
100
0
*
saline
missense
antisense

FIG. 4

TAIL FLICK

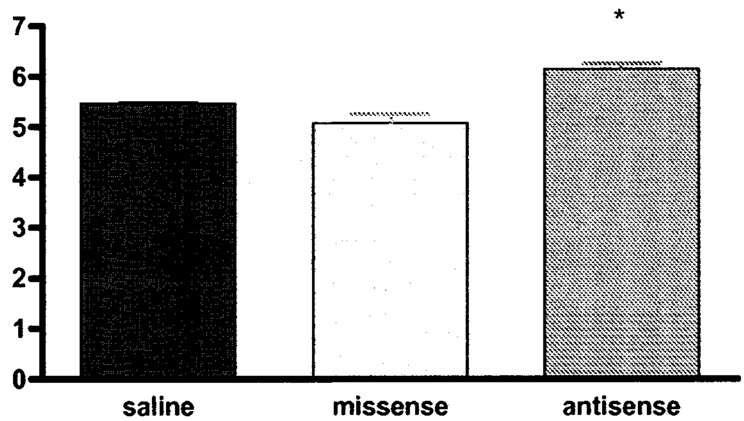


FIG. 5

# INTERNATIONAL SEARCH REPORT

International application No.
PCT/CN2006/000772

A. CLASSIFICATION OF SUBJECT MATTER

**See supplemental box**

According to International Patent Classification (IPC) or to both national classification and IPC

B. FIELDS SEARCHED

Minimum documentation searched (classification system followed by classification symbols)

A61K (2006.01), A61P (2006.01)

Documentation searched other than minimum documentation to the extent that such documents are included in the fields searched

Electronic data base consulted during the international search (name of data base and, where practicable, search terms used)

EPODOC,WPI,PAJ,BA,NCBI,CNPAT,CNKI

GPR39, GPR, G protein couple(d) receptor(s)

C. DOCUMENTS CONSIDERED TO BE RELEVANT

| Category* | Citation of document, with indication, where appropriate, of the relevant passages | Relevant to claim No. |
|---|---|---|
| X | WO 2004099782 A2 (FARB), 18.11.2004, see pages 40,45,55,60-61,75,77 and 79, claims 20,22, squence listing | 1-10 |
| A | Karen Kulju McKee et al: "Cloning and Characterization of Two Human G Protein-Coupled Receptor Genes (GPR38 and GPR39) Related to the Growth Hormone Secretagogue and Neurotensin Receptors", GENOMICS, 46(3), 1997, pp. 426-434 | 1-10 |
| A | LIU,Yongxue: "Orphan G protein-coupled receptors and their significance as novel drug targets", Chinese Pharmacological Bulletin, 19(6), 2003 Jun, pp. 601-604 | 1-10 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 14 Jul. 2006 (14.07.2006) | 10 · AUG 2006 (1 0 · 0 8 · 2 0 0 6) |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>LU, Chun<br>Telephone No. (86-10) 62085058 |

Form PCT/ISA /210 (second sheet) (April 2005)

## INTERNATIONAL SEARCH REPORT

International application No.
PCT/CN2006/000772

CLASSIFICATION OF SUBJECT MATTER

A61K38/17 (2006.01) i
A61P1/14 (2006.01) i
A61P3/04 (2006.01) i
A61P25/04 (2006.01) i

Form PCT/ISA /210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MCKEE KK ; TAN CP ; PALYHA OC ; LIU J ; FEIGHNER SD ; HRENIUK DL ; SMITH RG ; HOWARD AD ; VAN DER PLOEG LH.** Cloning and characterization of two human G protein-coupled receptor genes (GPR38 and GPR39) related to the growth hormone secretagogue and neurotensin receptors. *Genomics,* 15 December 1997, vol. 46 (3), 426-34
- **BIRGITTE HOLSTL ; NICHOLAS D.HOLLIDAY2 ; ANDERS BACH1 ; CHRISTIAN E.ELLING3 ; HELEN M.COX2 ; THUE W.SCHWARTZ1.** *J Biol Chem.,* 21 September 2004, vol. 3 Common
- **KOHLER et al.** *Eur. J. Immunol.,* 1976, vol. 6, 292
- **HAMMERLING et al.** In Monoclonal Antibodies and T Cell Hybridomas. Elsevier, 1981
- **SAMBROOK et al.** Molecular Cloning: A Laboratory Manual. Cold Spring Harbor Laboratory Press, 1989
- **AO, HONG.** Studies on four genes in mouse for their effects on food intake, water intake and protein metabolism. *Acta Laboratorium Animalis Scientia Sinica,* 2002, vol. 10 (1), 10-15

SEQUENCE LISTING

<110> SHANGHAI INSTITUTES FOR BIOLOGICAL SCIENCES, CAS

<120> FUNCTION AND USE OF GPR39 GENE IN MAMMMAL CENTRAL NERVOUS SYSTEM

<130> 052361

<150> CN 200510025323.7
<151> 2005-04-22

<160> 8

<170> PatentIn version 3.1

<210> 1
<211> 1371
<212> DNA
<213> Mus musculus

<400> 1
```
atggcttcat ccagtggctc caaccacatc tgctcccgtg tcatcgatca cagccatgtt      60
cctgaatttg aggtggccac ttggatcaaa atcaccctca tcttggtgta cctgatcatc     120
tttgtggtag gcatcttggg caacagcgtc accatcaggg ttacgcaggt attgcagaag     180
aagggctatt tgcagaagga ggtgacagat cacatggtca gtttggcttg ttcagatatc     240
ttggtctttt tgattggcat gcccatggag ttctacagca tcatttggaa ccccctgacc     300
acacccagct atgctctgtc ctgtaagctc cacacgttcc tctttgagac gtgcagctac     360
gccacactgc tgcacgtgct gaccctcagc tttgagcgct acattgccat ttgtcatccc     420
ttcaagtata aagcagtgtc tggacctcgc caggtgaaac tgctgattgg ctttgtatgg     480
gtcacctccg ccctggtggc actgcctttg ctctttgcca tgggtatcga gtaccctctg     540
gtaaacgtac ccactcacaa gggactcaac tgcaacctct ctcgcacccg ccaccacgat     600
gaacctggaa actccaatat gtccatctgc acgaacctct ccaaccgttg ggaggtcttc     660
cagtccagca tctttggggc ctttgctgtt tacctggtgg tcctggcgtc tgtggctttc     720
atgtgttgga atatgatgaa agtgctaatg aagagcaagc agggcactct tgcagggacc     780
gggccacagc tccagctgag gaagtcagag agtgaggaga gccggacagc aagaagacag     840
accatcatat tcctgagact gattgtggtg acgttggccg tgtgttggat gcccaatcag     900
atccgacgga tcatggctgc agcaaaaccc aaacatgact ggaccagaac gtacttcagg     960
gcatacatga tcctcctgcc cttctccgac accttcttct acctcagctc cgtggtcaac    1020
cctctcctct acaacgtgtc ctctcagcag ttccggaagg tgttctggca ggtgctctgc    1080
tgccgcctga ctctgcagca tgccaaccaa gagaaacgcc agcgtgcccg cttcatctcc    1140
accaaggaca gcaccagctc agcccgcagc cccctcatct tcctagcttc ccggcgcagt    1200
aactcttcct ccaggagaac taacaaggtt ttcttaagca cttttcagac tgaggccaag    1260
cctggagagg ctaagcccca gcccttgagt cctgagtcac cacagactgg ctcagagacc    1320
aaaccagctg ggtccaccac agaaaatagt ttacaggagc aggaagtatg a             1371
```

<210> 2
<211> 456
<212> PRT
<213> Mus musculus

<400> 2

```
Met Ala Ser Ser Ser Gly Ser Asn His Ile Cys Ser Arg Val Ile Asp
1               5                   10                  15
His Ser His Val Pro Glu Phe Glu Val Ala Thr Trp Ile Lys Ile Thr
            20                  25                  30
Leu Ile Leu Val Tyr Leu Ile Ile Phe Val Val Gly Ile Leu Gly Asn
        35                  40                  45
Ser Val Thr Ile Arg Val Thr Gln Val Leu Gln Lys Lys Gly Tyr Leu
    50                  55                  60
Gln Lys Glu Val Thr Asp His Met Val Ser Leu Ala Cys Ser Asp Ile
65                  70                  75                  80
Leu Val Phe Leu Ile Gly Met Pro Met Glu Phe Tyr Ser Ile Ile Trp
```

```
                85                  90                  95
Asn Pro Leu Thr Thr Pro Ser Tyr Ala Leu Ser Cys Lys Leu His Thr
            100                 105                 110
Phe Leu Phe Glu Thr Cys Ser Tyr Ala Thr Leu Leu His Val Leu Thr
        115                 120                 125
Leu Ser Phe Glu Arg Tyr Ile Ala Ile Cys His Pro Phe Lys Tyr Lys
    130                 135                 140
Ala Val Ser Gly Pro Arg Gln Val Lys Leu Leu Ile Gly Phe Val Trp
145                 150                 155                 160
Val Thr Ser Ala Leu Val Ala Leu Pro Leu Leu Phe Ala Met Gly Ile
                165                 170                 175
Glu Tyr Pro Leu Val Asn Val Pro Thr His Lys Gly Leu Asn Cys Asn
            180                 185                 190
Leu Ser Arg Thr Arg His His Asp Glu Pro Gly Asn Ser Asn Met Ser
        195                 200                 205
Ile Cys Thr Asn Leu Ser Asn Arg Trp Glu Val Phe Gln Ser Ser Ile
    210                 215                 220
Phe Gly Ala Phe Ala Val Tyr Leu Val Val Leu Ala Ser Val Ala Phe
225                 230                 235                 240
Met Cys Trp Asn Met Met Lys Val Leu Met Lys Ser Lys Gln Gly Thr
                245                 250                 255
Leu Ala Gly Thr Gly Pro Gln Leu Gln Leu Arg Lys Ser Glu Ser Glu
            260                 265                 270
Glu Ser Arg Thr Ala Arg Arg Gln Thr Ile Ile Phe Leu Arg Leu Ile
        275                 280                 285
Val Val Thr Leu Ala Val Cys Trp Met Pro Asn Gln Ile Arg Arg Ile
    290                 295                 300
Met Ala Ala Ala Lys Pro Lys His Asp Trp Thr Arg Thr Tyr Phe Arg
305                 310                 315                 320
Ala Tyr Met Ile Leu Leu Pro Phe Ser Asp Thr Phe Phe Tyr Leu Ser
                325                 330                 335
Ser Val Val Asn Pro Leu Leu Tyr Asn Val Ser Ser Gln Gln Phe Arg
            340                 345                 350
Lys Val Phe Trp Gln Val Leu Cys Cys Arg Leu Thr Leu Gln His Ala
        355                 360                 365
Asn Gln Glu Lys Arg Gln Arg Ala Arg Phe Ile Ser Thr Lys Asp Ser
    370                 375                 380
Thr Ser Ser Ala Arg Ser Pro Leu Ile Phe Leu Ala Ser Arg Arg Ser
385                 390                 395                 400
Asn Ser Ser Ser Arg Arg Thr Asn Lys Val Phe Leu Ser Thr Phe Gln
                405                 410                 415
Thr Glu Ala Lys Pro Gly Glu Ala Lys Pro Gln Pro Leu Ser Pro Glu
            420                 425                 430
Ser Pro Gln Thr Gly Ser Glu Thr Lys Pro Ala Gly Ser Thr Thr Glu
        435                 440                 445
Asn Ser Leu Gln Glu Gln Glu Val
    450                 455
```

<210> 3
<211> 1362
<212> DNA
<213> Homo sapiens

<400> 3

```
atggcttcac ccagcctccc gggcagtgac tgctcccaaa tcattgatca cagtcatgtc     60
cccgagtttg aggtggccac ctggatcaaa atcaccctta ttctggtgta cctgatcatc    120
ttcgtgatgg gccttctggg gaacagcgcc accattcggg tcacccaggt gctgcagaag    180
aaaggatact tgcagaagga ggtgacagac cacatggtga gtttggcttg ctcggacatc    240
ttggtgttcc tcatcggcat gcccatggag ttctacagca tcatctggaa tcccctgacc    300
acgtccagct acaccctgtc ctgcaagctg cacactttcc tcttcgaggc ctgcagctac    360
gctacgctgc tgcacgtgct gacactcagc tttgagcgct acatcgccat ctgtcacccc    420
ttcaggtaca aggctgtgtc gggaccttgc caggtgaagc tgctgattgg cttcgtctgg    480
```

```
gtcacctccg ccctggtggc actgcccttg ctgtttgcca tgggtactga gtacccccctg    540
gtgaacgtgc ccagccaccg gggtctcact tgcaaccgct ccagcacccg ccaccacgag    600
cagcccgaga cctccaatat gtccatctgt accaacctct ccagccgctg gaccgtgttc    660
cagtccagca tcttcggcgc cttcgtggtc tacctcgtgg tcctgctctc cgtagccttc    720
atgtgctgga acatgatgca ggtgctcatg aaaagccaga agggctcgct ggccgggggc    780
acgcggcctc cgcagctgag gaagtccgag agcgaagaga gcaggaccgc caggaggcag    840
accatcatct tcctgaggct gattgttgtg acattggccg tatgctggat gcccaaccag    900
attcggagga tcatggctgc ggccaaaccc aagcacgact ggacgaggtc ctacttccgg    960
gcgtacatga tcctcctccc cttctcggag acgtttttct acctcagctc ggtcatcaac   1020
ccgctcctgt acacggtgtc ctcgcagcag tttcggcggg tgttcgtgca ggtgctgtgc   1080
tgccgcctgt cgctgcagca cgccaaccac gagaagcgcc tgcgcgtaca tgcgcactcc   1140
accaccgaca gcgcccgctt tgtgcagcgc ccgttgctct tcgcgtcccg gcgccagtcc   1200
tctgcaagga gaactgagaa gatttcttta agcacttttc agagcgaggc cgagccccag   1260
tctaagtccc agtcattgag tctcgagtca ctagagccca actcaggcgc gaaaccagcc   1320
aattctgctg cagagaatgg ttttcaggag catgaagttt ga                      1362
```

<210> 4
<211> 453
<212> PRT
<213> Homo sapiens

<400> 4

```
Met Ala Ser Pro Ser Leu Pro Gly Ser Asp Cys Ser Gln Ile Ile Asp
1               5                   10                  15
His Ser His Val Pro Glu Phe Glu Val Ala Thr Trp Ile Lys Ile Thr
            20                  25                  30
Leu Ile Leu Val Tyr Leu Ile Ile Phe Val Met Gly Leu Leu Gly Asn
        35                  40                  45
Ser Ala Thr Ile Arg Val Thr Gln Val Leu Gln Lys Lys Gly Tyr Leu
    50                  55                  60
Gln Lys Glu Val Thr Asp His Met Val Ser Leu Ala Cys Ser Asp Ile
65                  70                  75                  80
Leu Val Phe Leu Ile Gly Met Pro Met Glu Phe Tyr Ser Ile Ile Trp
                85                  90                  95
Asn Pro Leu Thr Thr Ser Ser Tyr Thr Leu Ser Cys Lys Leu His Thr
            100                 105                 110
Phe Leu Phe Glu Ala Cys Ser Tyr Ala Thr Leu Leu His Val Leu Thr
        115                 120                 125
Leu Ser Phe Glu Arg Tyr Ile Ala Ile Cys His Pro Phe Arg Tyr Lys
    130                 135                 140
Ala Val Ser Gly Pro Cys Gln Val Lys Leu Leu Ile Gly Phe Val Trp
145                 150                 155                 160
Val Thr Ser Ala Leu Val Ala Leu Pro Leu Leu Phe Ala Met Gly Thr
                165                 170                 175
Glu Tyr Pro Leu Val Asn Val Pro Ser His Arg Gly Leu Thr Cys Asn
            180                 185                 190
Arg Ser Ser Thr Arg His His Glu Gln Pro Glu Thr Ser Asn Met Ser
        195                 200                 205
Ile Cys Thr Asn Leu Ser Ser Arg Trp Thr Val Phe Gln Ser Ser Ile
    210                 215                 220
Phe Gly Ala Phe Val Val Tyr Leu Val Val Leu Leu Ser Val Ala Phe
225                 230                 235                 240
Met Cys Trp Asn Met Met Gln Val Leu Met Lys Ser Gln Lys Gly Ser
                245                 250                 255
Leu Ala Gly Gly Thr Arg Pro Pro Gln Leu Arg Lys Ser Glu Ser Glu
            260                 265                 270
Glu Ser Arg Thr Ala Arg Arg Gln Thr Ile Ile Phe Leu Arg Leu Ile
        275                 280                 285
Val Val Thr Leu Ala Val Cys Trp Met Pro Asn Gln Ile Arg Arg Ile
    290                 295                 300
Met Ala Ala Ala Lys Pro Lys His Asp Trp Thr Arg Ser Tyr Phe Arg
305                 310                 315                 320
```

Ala Tyr Met Ile Leu Leu Pro Phe Ser Glu Thr Phe Phe Tyr Leu Ser
325 330 335
Ser Val Ile Asn Pro Leu Leu Tyr Thr Val Ser Ser Gln Gln Phe Arg
340 345 350
Arg Val Phe Val Gln Val Leu Cys Cys Arg Leu Ser Leu Gln His Ala
355 360 365
Asn His Glu Lys Arg Leu Arg Val His Ala His Ser Thr Thr Asp Ser
370 375 380
Ala Arg Phe Val Gln Arg Pro Leu Leu Phe Ala Ser Arg Arg Gln Ser
385 390 395 400
Ser Ala Arg Arg Thr Glu Lys Ile Phe Leu Ser Thr Phe Gln Ser Glu
405 410 415
Ala Glu Pro Gln Ser Lys Ser Gln Ser Leu Ser Leu Glu Ser Leu Glu
420 425 430
Pro Asn Ser Gly Ala Lys Pro Ala Asn Ser Ala Ala Glu Asn Gly Phe
435 440 445
Gln Glu His Glu Val
450

<210> 5
<211> 1371
<212> DNA
<213> rat

<400> 5
atggcttcat ccagtggctc cagcaacatc tgctcccgag tcatcgatca cagccatgtc 60
cctgagttcg aagtggccac ttggatcaaa atcaccctca ccttggtgta cctgatcgtc 120
ttcgtggtag gcatcttggg caatagcgtc accatccggg ttacgcaggt attgcagaaa 180
aagggctatt tgcagaagga ggtgacagat cacatgatca gtttggcttg ttcagatatc 240
ttggtctttt tgattggcat gcccatggag ttctacagca tcatctggaa ccccctgacc 300
acacccagct atgctctgtc ctgcaagctc cacacgttcc tctttgagac gtgtagctac 360
gccacattgc tgcatgtgct gaccctcagc tttgagcgct acattgccat ttgtcatccc 420
ttcagatata aggacgtgtc tggaccttgc caggtgaaac tgctgatcgg ctttgtatgg 480
gtcacctccg ctctggtggc actgcccttg ctctttgcca tgggtattga gtaccctctg 540
gcgaacgtcc ccactcacaa gggactcaac tgtaacctct ctcgtacccg ccaccacgat 600
catcctggag actccaatat gtccatctgc acgaacctct ccagccgttg ggaggtcttc 660
cagtccagca tctttggggc cttcgctgtt tacctggtgg tcctggtgtc tgtggctttc 720
atgtgttgga acatgatgaa agtgctaatg aagagcaagc ggggtactct ggcagggacc 780
ggaccacagc tgcagctgcg gaagtcagag agtgaggaga gccggacagc gagaagacag 840
accatcatat tcctgagact gatcgtggtg acactggccg tgtgttggat gccaaatcag 900
atccgacgga tcatggccgc agcaaaaccc aaacatgact ggaccaagtc gtacttcaag 960
gcgtacatga tcctcctccc cttctccgac accttcttct acctcagctc cgtggtcaac 1020
cctctcctct acaacgtgtc ttctcagcag ttccggaagg ttttctggca ggttctctgc 1080
tgccggctga ctctgcagca tgccaaccag gagaaacagc agcgtgccta cttcagctct 1140
accaaaaaca gcagccgctc agcccgaagc ccgctcatct tcctagcctc ccggcgtagt 1200
aactcttcct cccggagaac taacaaggtt ttcttaagca cttttcaggc ggaggctaag 1260
cctctagagg gcgagcacca gcccttgagt cctgagtcac cacagaccgg ctcagagacc 1320
aaacctgctg gttccgccac agaaaatagt ttacaggagc aggaagtgtg a 1371

<210> 6
<211> 456
<212> PRT
<213> rat

<400> 6

Met Ala Ser Ser Ser Gly Ser Ser Asn Ile Cys Ser Arg Val Ile Asp
1 5 10 15
His Ser His Val Pro Glu Phe Glu Val Ala Thr Trp Ile Lys Ile Thr
20 25 30
Leu Thr Leu Val Tyr Leu Ile Val Phe Val Val Gly Ile Leu Gly Asn

```
        35                  40                  45
Ser Val Thr Ile Arg Val Thr Gln Val Leu Gln Lys Lys Gly Tyr Leu
    50                  55                  60
Gln Lys Glu Val Thr Asp His Met Ile Ser Leu Ala Cys Ser Asp Ile
65                  70                  75                  80
Leu Val Phe Leu Ile Gly Met Pro Met Glu Phe Tyr Ser Ile Ile Trp
                85                  90                  95
Asn Pro Leu Thr Thr Pro Ser Tyr Ala Leu Ser Cys Lys Leu His Thr
            100                 105                 110
Phe Leu Phe Glu Thr Cys Ser Tyr Ala Thr Leu Leu His Val Leu Thr
        115                 120                 125
Leu Ser Phe Glu Arg Tyr Ile Ala Ile Cys His Pro Phe Arg Tyr Lys
    130                 135                 140
Asp Val Ser Gly Pro Cys Gln Val Lys Leu Leu Ile Gly Phe Val Trp
145                 150                 155                 160
Val Thr Ser Ala Leu Val Ala Leu Pro Leu Leu Phe Ala Met Gly Ile
                165                 170                 175
Glu Tyr Pro Leu Ala Asn Val Pro Thr His Lys Gly Leu Asn Cys Asn
            180                 185                 190
Leu Ser Arg Thr Arg His His Asp His Pro Gly Asp Ser Asn Met Ser
        195                 200                 205
Ile Cys Thr Asn Leu Ser Ser Arg Trp Glu Val Phe Gln Ser Ser Ile
    210                 215                 220
Phe Gly Ala Phe Ala Val Tyr Leu Val Val Leu Val Ser Val Ala Phe
225                 230                 235                 240
Met Cys Trp Asn Met Met Lys Val Leu Met Lys Ser Lys Arg Gly Thr
                245                 250                 255
Leu Ala Gly Thr Gly Pro Gln Leu Gln Leu Arg Lys Ser Glu Ser Glu
            260                 265                 270
Glu Ser Arg Thr Ala Arg Arg Gln Thr Ile Ile Phe Leu Arg Leu Ile
        275                 280                 285
Val Val Thr Leu Ala Val Cys Trp Met Pro Asn Gln Ile Arg Arg Ile
    290                 295                 300
Met Ala Ala Ala Lys Pro Lys His Asp Trp Thr Lys Ser Tyr Phe Lys
305                 310                 315                 320
Ala Tyr Met Ile Leu Leu Pro Phe Ser Asp Thr Phe Phe Tyr Leu Ser
                325                 330                 335
Ser Val Val Asn Pro Leu Leu Tyr Asn Val Ser Ser Gln Gln Phe Arg
            340                 345                 350
Lys Val Phe Trp Gln Val Leu Cys Cys Arg Leu Thr Leu Gln His Ala
        355                 360                 365
Asn Gln Glu Lys Gln Gln Arg Ala Tyr Phe Ser Ser Thr Lys Asn Ser
    370                 375                 380
Ser Arg Ser Ala Arg Ser Pro Leu Ile Phe Leu Ala Ser Arg Arg Ser
385                 390                 395                 400
Asn Ser Ser Ser Arg Arg Thr Asn Lys Val Phe Leu Ser Thr Phe Gln
                405                 410                 415
Ala Glu Ala Lys Pro Leu Glu Gly Glu His Gln Pro Leu Ser Pro Glu
            420                 425                 430
Ser Pro Gln Thr Gly Ser Glu Thr Lys Pro Ala Gly Ser Ala Thr Glu
        435                 440                 445
Asn Ser Leu Gln Glu Gln Glu Val
    450                 455
```

<210> 7
<211> 18
<212> DNA
<213> Artificial Sequence

<220>
<221> misc_feature
<223> Oligonucleotide

<400> 7
tcggatctga ttgggcat 18

<210> 8
<211> 18
<212> DNA
<213> Artificial Sequence

<220>
<221> misc_feature
<223> Oligonucleotide

<400> 8
ttgggtctga tcggacat 18